# EUROPEAN PATENT APPLICATION

(11) **EP 1 547 467 A1**
(43) Date of publication of application: **29.06.2005**
(21) Application number: 03447304.1
(22) Date of filing: 22.12.2003
(51) Int. Cl.: A21D 2/00, A21D 8/04, C12N 1/16

(54) **Liquid leaven composition**

(71) Applicant: PURATOS N.V., 1702 Groot-Bijgaarden (BE)
(72) Inventor: Bonjean, Bernard, 1495 Marbais (BE); Cappelle, Stefan, 9500 Onkerzele (BE); Dewilde, Christophe, 1640 Rhode-Saint-Genese (BE); Tossut, Pierre Patrick Aldo, 4350 Remicourt (BE)
(74) Representative: Van Malderen, Joelle

(57) **Abstract**

The present invention relates to a method of preparing a novel stable liquid leaven composition comprising a bread flavour improvement composition, an active yeast and a bread improver composition. Stable liquid compositions according to the invention advantageously are obtained when the residual sugar content is kept low, preferably below 0.5% w/w on the liquid composition and/or when severe drops in pH are prevented.

The present invention further relates to novel liquid leaven compositions obtained as such and to their use in for instance bakery products like bread, snacks and pizzas.

## Description

### Field of the invention

The present invention relates to novel stable liquid leaven compositions for bakery, snack (e.g. crackers, pretzels, biscuits, ...) and pizza applications comprising a bread flavour compound, an active yeast and a bread improver composition, to their use and their production.

### Background of the invention

Currently, a bread flavour enhancement system, a bread improver composition and the yeast are dosed each separately. There are several disadvantages coupled therewith.

Separate dosing increases the risk of faults as well as the labor costs incurred with said dosing. In case of liquid products, the investments in separate dosing are further significantly higher compared to a single dosing system since all piping, all pumps and automation has to be multiplied by the number of separate dosing points. Powdered dosing systems on the other hand are less accurate, or they require huge investments for automation. The dust production due to the dosing of powdered products is an increasing problem in the bakery due to its allergic properties. In terms of waste disposal, separate packaging will generate much more waste compared to an all-in-one solution. Separate products increase the number of stock keeping units, complicating the logistic organization of the bakery as well as a decreased working capital due to immobilization of capital into stocks.

A number of processes are known to improve the quality and flavour of the bread. Sourdough production and sponge production are two well known examples of fermentation systems that are produced in a bakery. The making of house-made sourdough implies a lot of organizational efforts as well as the risk of decreased consistency in the bakery. For these reasons, ready-to-use sourdough base compositions have been developed and marketed. Liquid sourdough base compositions have entered the market as bakery ingredients.

It is a new trend in bakery to supply the bread improvement system in liquid form. This bread improvement system comprises chemical additives (dough conditioners) such as oxidizing and reducing agents, emulsifiers, fatty materials and others, and enzymes.

Active yeast can be supplied to bakery as liquid product enabling accurate dosing, easier cleaning of the system etc Liquid yeast can be stabilized by adding hydrocolloids or gums such as xhantane gums or by continuous mixing (see EP-A-0461725). Alternatively, stabilization of the yeast can be obtained by using a 1% exopolysaccharide such as a dextran in the final product thereby preventing decantation as described in US Patent No. 6,399,119.

### Summary of the invention

It was surprisingly found that it was possible to combine or admix at least a bread flavour improvement composition, a bread improver composition and active yeast in a liquid formulation to obtain a liquid leaven composition which does not have the above-mentioned drawbacks and which is stable. As soon as there are traces of fermentable substrates introduced into liquid yeast, one would expect a refermentation of the yeast with instability during time as a consequence. Some of the bread flavour improvement systems contain alcohols which could be a potential carbon source, but the product stayed stable anyhow. Also the bread improver composition can be a source (N-source) for the yeast to start growing with both decreased activity of the improver and decreased gassing power of the yeast.

The novel liquid leaven composition according to the invention has the same gassing powder as fresh yeast, the dough and bread improvement properties of a regular bread improvement system and the flavour enhancement properties as one can achieve with a sourdough process or a sponge process.

Preferably this novel liquid leaven composition can be stored for a longer period at temperatures of about 4°C, for instance for at least one week, preferably for at least 4 weeks.

A liquid formulation is obtained by admixing the above ingredients with a liquid such as water and/or alcohols or glycerol for instance for enzyme stability. Alternatively one may combine each of the above ingredients in liquid form.

The present invention relates to a process for producing a liquid leaven composition comprising the step of admixing at least a flavour improvement composition, an improver composition and an active yeast in a liquid formulation, in such a way that the combined product is a stable liquid product having the combined properties of all 3 components separately.

Stability of the above product is hereby mainly obtained by keeping the residual sugar level of the composition low, preferably below 0.5% w/w on the liquid composition.

If needed, measures are taken to prevent a severe pH drop such as a drop of pH below 3.5 or below 4.0.

The flavour composition may be selected from the group consisting of a sourdough, a sourdough product, a sponge, a sponge product, a composition flavour (blend of aroma chemicals), an acid blend or blend of acidifying agents and/or any mixture thereof. The flavour composition may be flour based.

In an embodiment according to the invention, the residual sugar level is kept low by hydrolising the flour prior to fermentation to liberate fermentable sugars out of the starch.

In another embodiment according to the invention, the residual sugar level is kept low by using the supernatant of a liquid sourdough as flavour improver composition, said supernatant possibly being concentrated.

In still a further embodiment, the residual sugar level is kept low by using a sponge based flavour composition.

Alternatively, the residual sugar level may be kept low by using a composition not comprising fermentable sugars. Such composition may be selected from the group consisting of a blend of aroma chemicals, acids, acidifying agents and/or any mixture thereof.

The improver composition used may comprise chemical additives and/or enzymes.

Chemical additives may be selected from the group consisting of oxidizing/reducing agents (such as ascorbic acid), emulsifiers (such as DATEM, SSL, CSL, GMS, bile salts), fatty materials and any mixture thereof.

Enzymes may be selected from the group consisting of amylases, hemi-cellulases, oxidases, proteases, lipases and any mixture thereof.

A drop of the pH below 3.5 or below pH 4.0 may be prevented by adding a buffering system to the flavour composition, by controlling the pH and/or by selecting specific bacterial strains.

Preferably fresh yeast is used in the novel and inventive leaven composition according to the invention.

The yeast may be used under the form of compressed yeast with a dry matter of around 30% and/or may be used under the form of liquid yeast, preferably with a dry matter below 25%.

The liquid leaven composition may be further stabilised by adding a solution comprising a hydrocolloid or a gum, preferably a xanthane gum to the composition and/or by continuous mixing of the composition to prevent decantation.

Alternatively, the liquid leaven composition may be stabilised by using a 1% level of an exopolysaccharide such as a dextran in the final product thereby preventing decantation.

The invention further relates to a liquid leaven composition obtainable by a method according to any of the preceding claims.

The novel and inventive liquid leaven composition according to the invention preferably remains stable when stored for a longer period at low temperatures such as 4°C. Preferably the product remains stable for at least one week at these temperatures, more preferably for at least 4 weeks.

The present invention also relates to a dough, a bakery product, a pizza or a snack (e.g. crackers, pretzels, biscuits, ...) comprising the liquid leaven composition according to the present invention.

The present invention further concerns the use of the liquid leaven composition of the invention in the preparation process of a bakery product such as a bread, a pizza or a snack (e.g. crackers, pretzels, biscuits, ...).

### Brief description of the figures

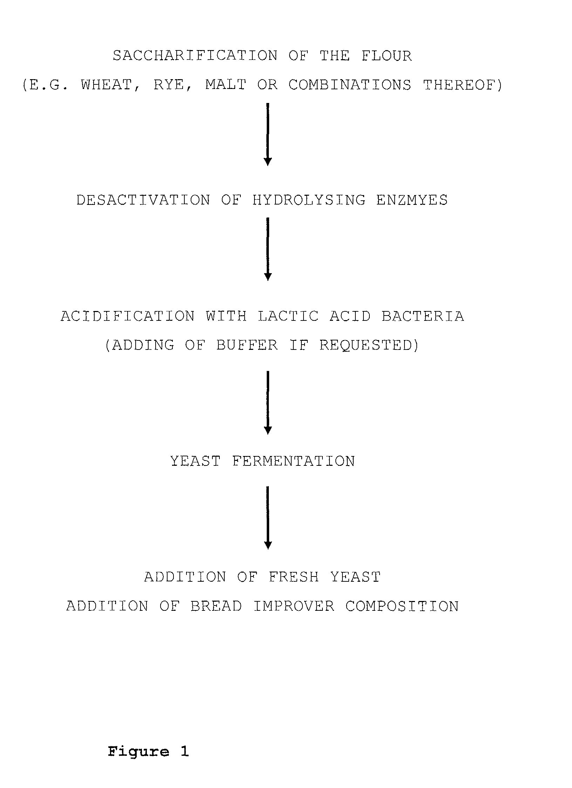

The figure 1 represents a schematic overview of a method for producing a stable liquid leaven composition according to the invention, wherein the amount of residual sugar is kept low.

The invention will be described in further details in the following examples by reference to the enclosed drawings, which are not in any way intended to limit the scope of the invention as claimed.

### Detailed description of the invention

Throughout this specification the following terms and definitions are used:

A "flavour improvement system" or a "bread flavour improvement system" or a "bread flavour improvement composition" refers to a sourdough or a sourdough product; a bakery sponge or a sponge product; or an other bread flavour improvement composition (see below).

By a "sourdough" is meant a dough fermented by lactic acid bacteria and/or yeast, having a characteristic acidic flavour due to the lactic acid bacteria producing mainly lactic acid, acetic acid and some minor compounds and the typical flavour top-notes produced by the yeast.

A "sourdough product" in the present context refers to the product above, that is stabilized in one or another way (e.g. through drying, pasteurization; cooling, freezing, ...) so that this product can be added to a regular dough, thereby replacing the in-bakery produced pre-fermentation.

By a "sponge" or "sponge dough" is meant a dough fermented by yeast, having a characteristic flavour due to said yeast fermentation. It is a pre-fermentation product based on a yeast fermentation of part of the flour.

A "sponge product" refers to the stabilized form of such a regular bakery sponge fermentation, used to enhance the flavour in a regular dough. It can be a sponge extract.

"Other flavour improver compositions" or "other bread flavour improver compositions" can be a blend of chemical aroma compounds and/or acids and/or acidifying agents (producing acid and/or gas).

"Improvement systems" or "bread improvement systems" or "(bread) improver compositions" may comprise chemical additives as well as enzymes, which are added to the dough in order to improve dough handling properties and/or quality of the final baked product. Chemical additives comprise oxidizing/reducing agents, emulsifiers, fatty materials and others. There is a wide range of enzymes that are used for bread improvement purposes. These enzymes are all well known and described in literature.

"Liquid yeast" corresponds to a new trend in bakery and is active yeast having a dry matter up to 25%, and often stabilized with hydrocolloids (see e.g. EP-A-0461725).

The present invention relates to the production process for such a combined product, resulting in a liquid leaven composition that improves the flavour of the final bread similarly to a bread made with the components of the bread improver composition each dosed separately. In the liquid composition according to the present invention, the yeast is sufficiently stable and shows a gassing production capacity comparable to any regular liquid yeast. The bread improver composition in this novel and inventive composition is sufficiently stable and results in dough and/or bread improvement properties comparable to those obtained when the components are each dosed separately.

To ensure stability of the complex flavour and leaven system of the present invention, the amount of fermentable substrates is preferably kept as low as possible. In sequel it is further explained that a normal freshly made sourdough based on regular flour without any processing could not be used as such because of flour components herein. A sponge which contains ethanol in amounts up to 10% seemed not to be a problem for stability as long as there were no flour traces anymore. In the case that a sourdough is being used in the liquid leaven composition, control of the pH may be needed. Below, more details and some examples are given on how the problem of stability of the product could be solved.

For the stability of the yeast within a (bread) flavour improvement composition, the residual sugar content should be as low as possible, optimally below 0.5%. Hereby a refermentation by the yeast in the packaging is prevented, which would otherwise result in severe foaming problems and cause instability of the yeast. The elimination of most fermentable sugars in a sourdough product can be obtained by different ways. Hydrolysis of the flour prior to fermentation is a first option. The fermentable sugars produced can be consumed in a sourdough or sponge process generating all necessary flavour compounds and limiting residual sugars.

A second solution to the problem consists of using only supernatant of a liquid sourdough as flavour improver composition, preventing hereby high amounts of starch into the liquid leaven composition. Starch, if present, could be further hydrolysed by microbial activity during storage causing gas production and instability.

A third way of reducing the residual sugar content consists of using the same compositions as mentioned above, but based on a sponge dough. No acidic flavour profile will be produced in this case. Other bread flavour compositions, not comprising fermentable sugars, can be used as well, said compositions comprising a blend of aroma chemicals, acids, acidifying agents, or a blend of the above products.

In order to stabilise the bread improver composition, the pH should not drop too much during sourdough production (i.e. the pH should preferably not drop below pH 3). In the case that enzymes are being used, such as e.g. amylase, the buffer capacity should be optionally improved in the sourdough production preventing a severe drop in pH. The pH should in this case preferably not fall beneath pH 3.5, optimally not beneath pH 4, to prevent loss of activity of the bread improvement system. The use of special lactic acid bacteria not acidifying beneath pH 3.5 is a possibility. This can easily be checked following pH profile during acidification using a pH-meter. Residual sugar will at that time be consumed by the yeast present during flavour formation.

It was surprisingly found that when respecting the above conditions, i.e. by keeping the residual sugar content low and/or by preventing severe and undesired pH drops, a stable liquid leaven could be obtained showing properties comparable to those obtained when each of the components would have been dosed separately.

### Examples

In the examples, the following materials and methods were used.

A typical yeast sponge dough was produced by fermenting flour slurry with yeast during several hours. The liquid supernatant is separated from the insoluble part and concentrated by physical process. The residual sugar level is below 0.5%.

Alternatively, a sourdough product was produced using a special flour extraction method as shown in figure 1.

A typical bread improvement system consisted of/comprised:
Vitamine C : Ascorbic acid
Bel'ase A75: fungal amylase (BELDEM, Belgium)
Bel'ase B210: bacterial xylanase (BELDEM, Belgium)
Bel'ase XL1: phospholipase (BELDEM, Belgium)

Compressed fresh yeast with a 30% dry matter content or alternatively, liquid yeast with a dry matter content of max. 25% and kept in suspension by continuous mixing, was used.

### Example 1

Liquid sourdough was produced according to a scheme as presented in Figure 1. A first step comprised saccharification of the flour (wheat, rye, malt, ... or any combination thereof) following the here-described hydrolysis protocol.

*Hydrolysis protocol:* Flour, which can be regular wheat flour, but also other types of flour such as rye, malt, corn,... flour and mixtures of them are added to water to obtain a flour slurry. The optimal dry matter content of this slurry is between 20 and 50% and more particular between 30 and 35%. The slurry is then heated, optimally to temperatures between 75 and 95°C, more particular to a temperature about 90°C ± 2% and the pH is adjusted to a pH of 5.5 ± 0.5. The slurry is maintained under constant mixture. An amylase is added, eg Thermamyl SC® (Novozymes, DK), at a dose of 0.4-0.5kg/ton of dry matter. Other amylases may be used. During the process of hydrolysis, the temperature is maintained at about 90°C and in the case of Thermamyl SC®, the reaction time was about 90 minutes. The reaction time will be dependent on the substrate and enzymes used, and a person skilled in the art will know how to adapt the described protocol accordingly.

After this hydrolysis step, the temperature is lowered, the pH adjusted and a dextrinisation can take place adding a second enzyme cutting the oligodextrines into fermentable sugars. In this particular case, dextrozyme GA (0.8kg/T dry matter) (Novozymes, DK) was used at a temperature of about 60°C and at a pH of about 4.3. This second hydrolysis was done during 22 hours. Dependent on the substrate and the enzyme, these conditions can be adapted by the person skilled in the art.

After a step of hydrolysis and deactivation of the hydrolysing enzymes, an acidification step as decribed below was performed.

*Acidification step:* After saccharification of the flour, lactic acid bacteria are added. The slurry is diluted with water before fermentation. In this example, the slurry was diluted to obtain a sugar content of around 10%. This slurry is inoculated with a lactic acid bacterium (*Lactobacillus plantarum* sp) at a rate of 10 E+7/g of dry matter. The acidification takes place during about 24 hours at about 35°C. These fermentation conditions can be adapted according to the strain used and the flavour profile aiming for. Colder fermentation conditions generally will produce more acetic acid, whereas warmer temperatures more lactic acid.

To ferment residual sugars after the acidification step, some yeast is added. This yeast will consume the sugar to values lower then 0.5% assuring the stability of the mixture afterwards.

After this step, the product is inactivated by heating the product, in the present case obtained by about 30 minutes heating at about 85°C.

To the above fermentation product, standard liquid yeast can now be added.

The composition of the bread improvement used in this first example is given in Table 1 below:

**Table 1**

| **Compound** | **Proportion** |
|---|---|
| Vit C | 5 |
| FRIMASE 210 (BELDEM, Belgium) | 3 |
| Amylase A75 (BELDEM, Belgium) | 1.5 |
| Bel'ase XL1 (BELDEM, Belgium) | 2.5 |
| Flour | 488 |
| Total | 500 |

The Dosage level used was 0.5% on total flour weight.

A bread was then baked following the recipe described below. In this first example, a comparison was made between a bread that that was prepared using the novel leaven composition of the invention (recipe 3) versus a bread whereby all the different components were dosed separately (recipes 1 and 2). Both the use of separately dosed compressed and liquid yeast was compared. Table 2 gives an overview of the components and their amounts used in the bread making process.

**Table 2**

| **(grams or % on total flour weight)** | | | |
|---|---|---|---|
| **Recipe** | **1** | **2** | **3** |
| Wheat flour (25°) | 2000 | 2000 | 2000 |
| H₂O ($) | 1100 | 1080 | 1080 |
| **Compressed yeast** | **40** | **---** | **--** |
| **Liquid yeast** | **---** | **66** | **--** |
| Salt | 40 | 40 | 40 |
| **Bread improvement (1)** **system** | **0.5%** | **0.5%** | |
| **Sourdough product** | **1.65%** | **1.65%** | |
| **New leaven composition (2)** | | | **5%** |
| (1): The composition of the bread improver composition is given in table 1. | | | |
| (2): The composition of the liquid leaven composition according to the invention is given in Table 3 below. | | | |
| ($): The process conditions applied were those given in Table 4 below. | | | |

**Table 3**

| | g/100kg bloem |
|---|---|
| Bel'ase B 210 (BELDEM, Belgium) | 3.0 |
| Bel'ase A75 (BELDEM, Belgium) | 1.5 |
| Vitamine C | 5.0 |
| Bel4ase XL1 (BELDEM, Belgium) | 2.5 |
| Sourdough product based on rye flour | 1650 |
| Liquid Yeast | 3338 |
| Total | 5000.0 |

**Table 4**

| | | | |
|---|---|---|---|
| Mixing time: Eberhardt N24 | 2'+4'30" | 2'+4'30" | 2'+4'30" |
| Temperature - H₂O (°C) | 18° | 18° | 18° |
| Temperature - dough (°C) | 29.4° | 29.5° | 29.6° |

The following observations were made when the bread was prepared with a liquid leaven composition that was stored for 1 week before use (Table 5). A comparison was made with a bread made using conventional ingredients, i.e. with each of the components dosed separately (recipes 1 and 2). The rating/scoring was done by a technician skilled in the art.

Table 6 summarizes the results of a test performed with a liquid leaven composition comprising flavour composition, liquid yeast and improvement composition that was maintained at 4°C for 4 weeks. After 4 weeks, it was baked again without loss of activity. Again a comparison was made with traditional recipes for bread making (recipes 1 and 2).

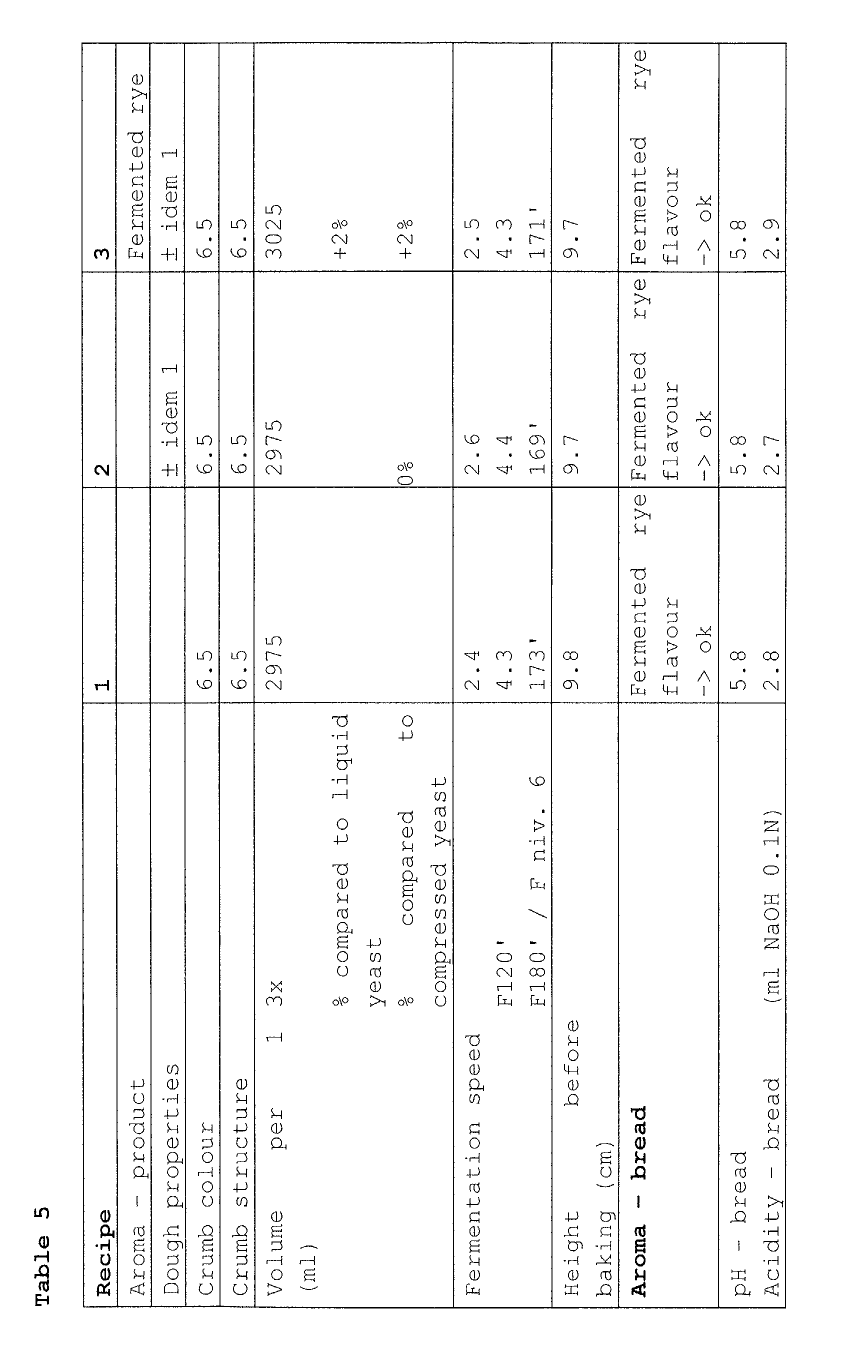

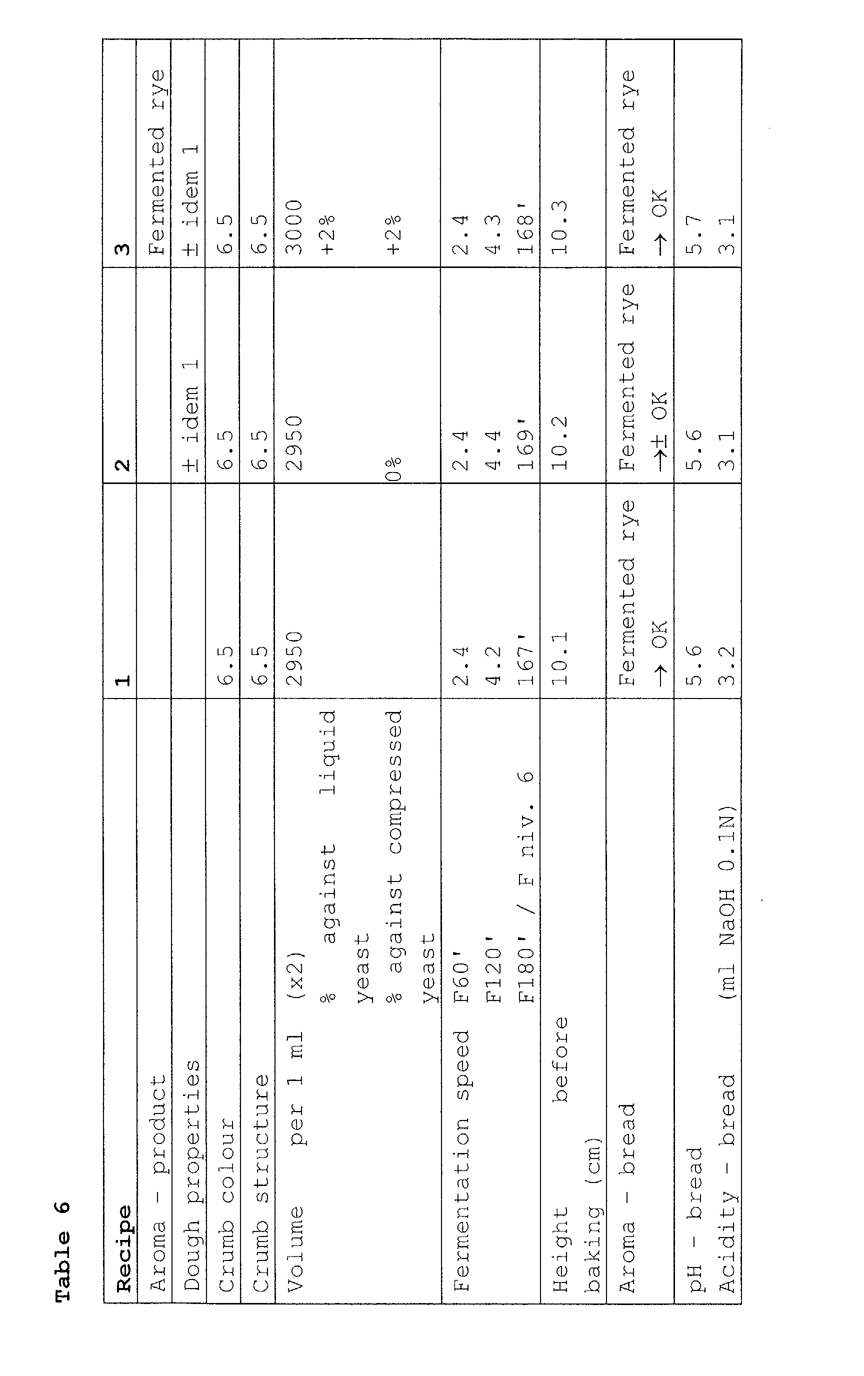

From the above it can be derived that the novel liquid leaven composition is very stable even after storage of 4 weeks at 4°C. Dough and bread properties remained unchanged in comparison with the separately dosed compounds (recipes 1 and 2). Independent of the fact whether compressed or liquid yeast was used, the leaven composition was found stable.

### Example 2:

In a second example, a bread was made according to two different recipes: one with each of the components dosed separately (recipe 1) and one with the novel liquid leaven composition (recipe 2) (Table 7).

**Table 7**

| **(grams or % on total flour weight)** | | |
|---|---|---|
| **Recipe** | **1** | **2** |
| Wheat flour (25°) | 2000 | 2000 |
| H₂O ($) | 1120 | 1120 |
| **Yeast** | **40** | **--** |
| Salt | 40 | 40 |
| **Bread improvement system** (1) | **0.5%** | |
| **Sponge extract** | **2%** | |
| **New liquid leaven composition** (3) | | **4%** |

The composition of the bread improvement system (1) is the same as described in Example 1 above.

The composition of the liquid leaven composition (3) used in recipe (2) is given in Table 8 below.

**Table 8**

| **Component** | **Proportion** |
|---|---|
| Vit C | 5 |
| Bel'ase B210 (BELDEM, Belgium) | 3 |
| Bel'ase A75 (BELDEM, Belgium) | 1.5 |
| Bel'ase XL1 (BELDEM, Belgium) | 2.5 |
| K₂HPO₄ | 16 |
| Sponge extract | 2000 |
| Yeast | 1972 |
| Total | 4000 |

The process conditions ($) that were applied are summarized in Table 9.

**Table 9**

| | | |
|---|---|---|
| Mixing time : Eberhardt N24 | 2'+4'30" | 2'+4'30" |
| Temperature - H₂O (°C) | 18° | 18° |
| Temperature - dough (°C) | 30.1° | 29.9° |

Again, the liquid leaven composition that was used in the bread making recipe 2 was used after 1 week, respectively 4 weeks of storage. The results are summarized in Tables 10 and 11 respectively.

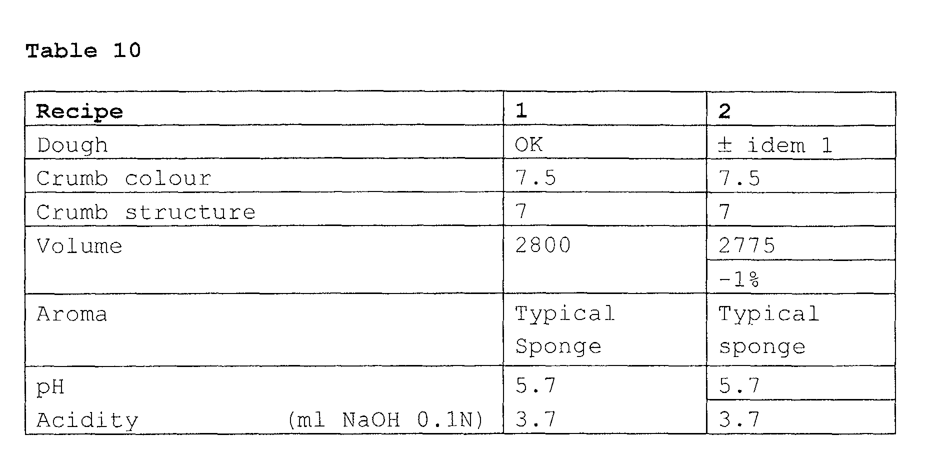

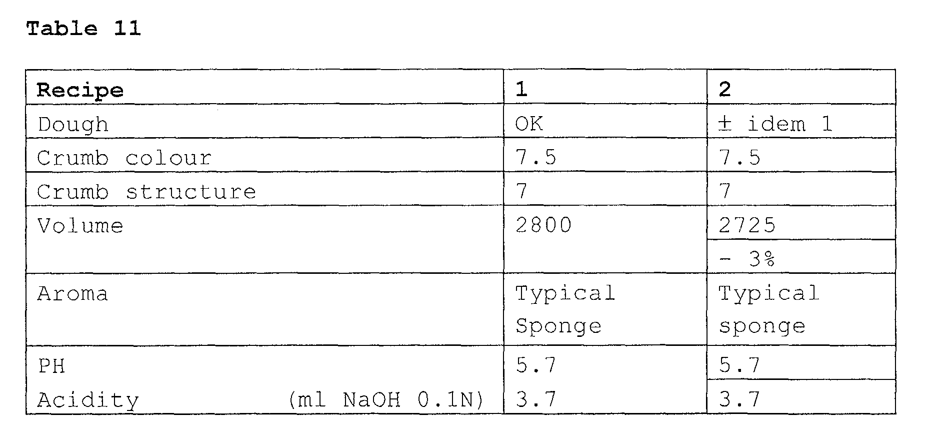

Also this example demonstrates that the liquid leaven composition according to the invention comprising a sponge extract, fresh yeast and a bread improver composition is stable for 4 weeks at 4°C.

## Claims

1. A process for producing a liquid leaven composition comprising the step of admixing at least a flavour improvement composition, an improver composition and an active yeast in a liquid formulation, wherein the residual sugar level of the liquid leaven composition is kept low, preferably below 0.5% w/w on the liquid composition to obtain a stable product.

2. The process according to claims 1, wherein additionally a drop of pH below 3.5, preferably below 4.0 is prevented.

3. The process according to any of the preceding claims, wherein the flavour composition is selected from the group consisting of a sourdough, a sourdough product, a sponge, a sponge product, a composition flavour (blend of aroma chemicals), an acid blend or blend of acidifying agents and any mixture thereof.

4. The process according to claim 3, wherein the flavour composition is flour based.

5. The process according to any of the preceding claims, wherein the residual sugar level is kept low by hydrolising the flour prior to fermentation to liberate fermentable sugars out of the starch.

6. The process according to any of the preceding claims, wherein the residual sugar level is kept low by using the supernatant of a liquid sourdough as flavour improvement composition, said supernatant possibly being concentrated.

7. The process according to any of the preceding claims, wherein the residual sugar level is kept low by using a sponge based flavour composition.

8. The process according to any of the preceding claims, wherein the residual sugar level is kept low by using a composition not comprising fermentable sugars.

9. The process according to claim 8, wherein said composition is selected from the group consisting of a blend of aroma chemicals, acids, acidifying agents and any mixture thereof.

10. The process according to any of the preceding claims wherein the improver composition comprises chemical additives and/or enzymes.

11. The process according to claim 10 wherein said chemical additives are selected from the group consisting of oxidizing/reducing agents (such as ascorbic acid), emulsifiers (such as DATEM, SSL, CSL, GMS, bile salts), fatty materials and any mixture thereof.

12. The process according to claim 10 wherein said enzymes are selected from the group consisting of amylases, hemi-cellulases, oxidases, proteases, lipases and any mixture thereof.

13. The process according to any of claims 2 to 12 wherein a drop of the pH below 3.5, preferably below pH 4.0 is prevented by adding a buffering system to the flavour composition, by controlling the pH and/or by selecting specific bacterial strains.

14. A process according to any of the preceding claims wherein fresh yeast is used.

15. The process according to claim 14 wherein the yeast is used under the form of compressed yeast with a dry matter of around 30% and/or under the form of liquid yeast, preferably with a dry matter below 25%.

16. The process according to any of the preceding claims wherein the liquid leaven composition is stabilised by adding a solution comprising a hydrocolloid or a gum, preferably a xanthane gum to the composition and/or by continuous mixing of the composition to prevent decantation.

17. The process according to any of the claims 1 to 15 wherein the liquid leaven composition is stabilised by using a 1% level of an exopolysaccharide such as a dextran in the final product thereby preventing decantation.

18. A liquid leaven composition obtainable by a method according to any of the preceding claims.

19. The product according to claims 18 which remains stable when stored for a longer period, preferably at least 1 week, most preferably at least 4 weeks, at 4°C.

20. A dough, a bakery product, a pizza or a snack comprising the liquid leaven composition according to claim 18 or 19.

21. The use of the liquid leaven composition according to claim 18 or 19 in the preparation process of a bakery product such as a bread, a pizza or a snack.
